# EUROPEAN PATENT SPECIFICATION

(11) **EP 3 035 987 B1**
(45) Date of publication and mention of the grant of the patent: **14.11.2018**
(21) Application number: 14795868.0
(22) Date of filing: 21.08.2014
(51) Int. Cl.: A61M 15/00, A61M 11/00, G06F 19/00

(54) **CONTROLLING A MEDICATION NEBULIZER THROUGH A SMARTPHONE**
STEUERUNG EINES MEDIZINISCHEN VERNEBLERS DURCH EIN SMARTPHONE
COMMANDE D'UN NÉBULISEUR DE MÉDICAMENT PAR L'INTERMÉDIAIRE D'UN TÉLÉPHONE INTELLIGENT

(30) Priority: 23.08.2013 US 201361869341 P
(43) Date of publication of application: 29.06.2016
(73) Proprietor: Koninklijke Philips N.V., 5656 AE Eindhoven (NL)
(72) Inventor: MORRISON, Mark, Steven, 5656 AE Eindhoven (NL); VON HOLLEN, Dirk, Ernest, 5656 AE Eindhoven (NL)
(74) Representative: Recoules, Hector
(86) International application number: PCT/IB2014/064001
(87) International publication number: WO 2015/025290

(56) References cited:
- WO-A1-2013/072863
- GB-A- 2 395 437
- US-A- 6 152 130
- US-A1- 2006 237 001

## Description

### BACKGROUND

### 1. Field

The present disclosure pertains to systems and methods that provide power and/or control for respiratory drug delivery devices, and, in particular, to operate a medication nebulizer, at least in part, through a portable computing device such as a smartphone.

### 2. Description of the Related Art

Respiratory drug delivery devices are used to treat many types of patients. Some types of respiratory drug delivery devices, for example nebulizers, include components that move at frequencies in the ultrasonic range. Device performance may depend on controlling such components with sufficient accuracy and efficacy. Positive treatment outcomes depend on many factors, including patient adherence. GB 2 395 437 discloses an inhalation apparatus. An airflow device is provided to detect the commencement of inhalation. Upon detection of commencement of inhalation an electronic control will cause the ultrasonic transducer to vibrate. WO 2013/072863A1 relates to a nebulizer, which comprises an oscillator which is amplified by a power amplifier. US 6 152 130 A relates to an inhalation device with acoustic control. A microphone is used to determine the presence or absence of a sufficiently strong sound in a binary fashion.

### SUMMARY

Accordingly, one or more embodiments of the present invention provide a system configured to deliver medicament to a subject according to claim 1. The system comprises a power subsystem, a respiratory medicament delivery device, and a sound input conversion subsystem. The power subsystem is configured to receive an input signal and provide power based on the received input signal. The respiratory medicament delivery device includes a vibrating element that is driven by the power provided by the power subsystem. The respiratory medicament delivery device is configured to deliver medicament, responsive to nebulization of the medicament by the vibrating element, to an airway of a subject. The sound input conversion subsystem is configured to receive an audio input signal. The sound input conversion subsystem is further configured to convert the audio input signal to an ultrasonic output signal. The vibrating element is controlled based on the ultrasonic output signal.

These and other aspects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of any limits.

### BRIEF DESCRIPTION OF THE DRAWINGS

FIGs. 1 and 3 schematically illustrate systems configured to deliver medicament to a subject, in accordance with one or more embodiments described in this disclosure; and
FIG. 2 illustrates a method of delivering medicament to a subject in accordance with one or more examples described in this disclosure.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, *i.e*., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (*i.e*., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

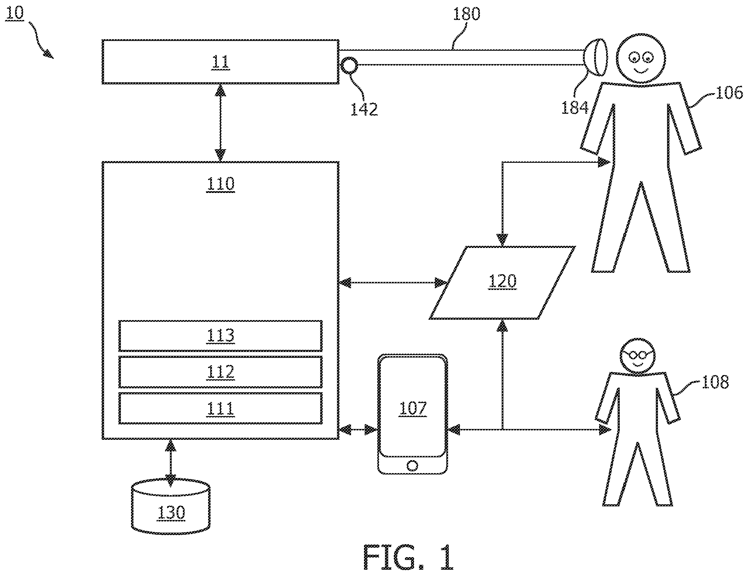

FIG. 1 schematically illustrates a system 10 configured to deliver medicament to a subject 106. System 10 may include one or more of a respiratory medicament delivery device 11, one or more sensors 142, one or more processors 110, a parameter determination module 111, a control module 112, an adherence module 113, an electronic storage 130, a user interface 120, a computing device 107, and/or other components and/or computer program modules.

Respiratory medicament delivery device 11 may be one or more of a jet nebulizer, a mesh nebulizer, an ultrasonic wave nebulizer, a nebulizer, an aerosol generator, and/or another device configured to deliver medicament to a subject through, at least in part, respiration of the subject. In some implementations, respiratory medicament delivery device 11 may include one or more features of one or more of these devices. Respiratory medicament delivery device 11 may be configured to combine breathable gas, *e.g.* air, and medicament, *e.g.* liquid and/or aerosolized drugs, for delivery to the airway of subject 106.

Respiratory medicament delivery device 11 may emit energy during operation, including, but not limited to, ultrasonic energy and/or energy in the audible part of the spectrum. As used herein, the audible part of the spectrum may be used to refer to frequencies below about 20 kHz. Respiratory medicament delivery device 11 may be configured such that a constituent component of respiratory medicament delivery device 11 displaces air and/or gas through mechanical movement at an ultrasonic frequency. Such displacement may be indirect, *e.g.* when a moving component is coupled to another component which transfers energy to air and/or gas. In some implementations, respiratory medicament delivery device 11 may emit energy in a frequency range between about 18 kHz and about 200 kHz, and/or any sub-range thereof. The specific frequency range may depend on the type of respiratory medicament delivery device that is used. For example, mesh nebulizers may include one or more vibrating elements that operate and emit ultrasonic energy at frequencies of about 100 kHz, between about 100 kHz and about 130 kHz, between about 120 kHz and 200 kHz, and/or other suitable frequencies. In some implementations, nebulizers may operate at frequencies of 1 MHz or more.

Computing device 107 may include one or more of a headphone connector, a universal serial bus (USB) connector, a docking connector, and/or other standardized connectors commonly used in the fields of portable consumer electronics. In some implementations, computing device 107 may include a power source, including but not limited to a (rechargeable) battery. In some implementations, computing device 107 may include information processing capabilities. By way of non-limiting example, computing device 107 may include one or more of a desktop computer, a laptop computer, a handheld computer, a NetBook, a tablet, a smartphone, a wireless telephone, a portable electronic device, a handheld communication device, a digital camera, a gaming console, and/or other computing platform(s).

By virtue of this disclosure, one or more of power and/or control for respiratory medicament delivery device 11 may be provided by and/or through computing device 107. In some implementations, computing device 107 may be configured to provide power to respiratory medicament delivery device 11. In some implementations, computing device 107 may be configured to provide one or more control functions for the operation of respiratory medicament delivery device 11. In some implementations, computing device 107 may be configured to provide (adherence) monitoring for respiratory medicament delivery device 11. Provision of one or more functions external to respiratory medicament delivery device 11 may allow a reduction in complexity, components, and/or materials for respiratory medicament delivery device 11. For example, in some implementations, respiratory medicament delivery device 11 may not need to include a battery or other type of power source, thereby simplifying the design and manufacture of respiratory medicament delivery device 11.

In some implementations, respiratory medicament delivery device 11 may be operated, at least in part, by care provider 108, *e.g.* a medical professional. In some implementations, respiratory medicament delivery device 11 may be operated, at least in part, through computing device 107, *e.g.* through a software application running on the device.

In some implementations, respiratory medicament delivery device 11 may include a conduit 180 to guide gas and/or medicament to subject 106 and/or a mouthpiece 184 to deliver gas and/or medicament from conduit 180 to the airway of subject 106.

In some implementations, respiratory medicament delivery device 11 may include a mesh nebulizer and/or components/features thereof. In some implementations, respiratory medicament delivery device 11 may include an ultrasonic wave nebulizer and/or components/features thereof. In some implementations, respiratory medicament delivery device 11 may include an aerosol generator and/or components/features thereof. Mesh nebulizers, ultrasonic wave nebulizers, aerosol generators, and/or combinations thereof may include one or more piezoelectric elements to provide mechanical vibration and thus displacement of a medium, *e.g.* liquid or air. Nebulizers filled with liquid may include moving components that transfer ultrasonic energy to air and/or gas. In some implementations, one or more other surfaces in direct contact with air and/or gas may move as a result of the motion of, *e.g.,* a piezoelectric element. Any vibrating surface may emit ultrasonic energy. For example, the backside of a piezoelectric element may contact (and/or be coupled with) air and/or gas. In some implementations, the piezoelectric element is coupled with a mesh (*e.g.* a mesh nebulizer) having a side that is directly (or indirectly) in contact with air and/or gas. In some implementations, a static mesh may be placed at some harmonic distance from a vibrating piezoelectric element.

Piezoelectric elements may achieve maximum displacement at one or more particular frequencies, referred to as resonant frequencies. Maximum displacement may be targeted as a preferred mode of operation (and/or the operating frequency). The operating frequency may characterize operation of the piezoelectric element and/or respiratory medicament delivery device 11. Operating conditions and/or maximum displacement may change over time, *e.g.* depending on the amount of available medicament within the device, the loading, drift of an oscillator used with/within the device, wear and tear of the device, ambient operating conditions such as temperature, humidity, atmospheric pressure, air density, and/or other factors that may change over time. Operating conditions and/or maximum displacement may differ between individual devices, *e.g.* based on construction, assembly, and/or other device-specific conditions. The particular operating condition that has maximum displacement may be assumed to coincide, or at least be close to, the operating condition in which respiratory medicament delivery device 11 emits a maximum amount of ultrasonic energy. As used herein, the term "maximum" may refer to a local maximum in a specific range of operation.

Operational parameters for respiratory medicament delivery device 11 may be adjusted to track changes in (maximum) displacement, operating conditions, emitted (ultrasonic) energy, measured parameters, and/or other changes. Adjustments may be based on (feedback of) measurements of ultrasonic energy emitted by respiratory medicament delivery device 11, *e.g.* in a breath-actuated mode of operation. In some implementations, adjustments may be made in real-time or near-real-time. In some implementations, adjustments may be made automatically, autonomously, and/or without manual user intervention. In some implementations, respiratory medicament delivery device 11 may include an electronic oscillator or similar device/component to control the driving frequency of the piezoelectric element and/or other component configured for intentional displacement of, *e.g.,* a medium.

One or more sensors 142 of system 10 in FIG. 1 are configured to generate output signals representing one or more characteristics of ultrasonic energy emitted by respiratory medicament delivery device 11. In some implementations, sensor 142 may include a microphone (interchangeably referred to as microphone 142). For example, sensor 142 may include a microphone constructed as a micro-electro-mechanical system (MEMS) or nano-electro-mechanical system (NEMS). As used herein, the term "MEMS" may be used to refer to either MEMS or NEMS. As used in this disclosure, the term "microphone" may be used to refer to a MEMS microphone, and may be used for audible and/or ultrasonic frequencies/sounds.

The one or more sensors 142 may include an accelerometer, positional sensor, movement sensor, light sensor, infra-red (IR) sensor, electromagnetic sensor, electrode, tilt meter, (video) camera, and/or other sensors. The illustration of sensor 142 including one member in FIG. 1 is not intended to be limiting. In some embodiments, system 10 may use multiple sensors. The illustration of the location of sensor 142 as depicted in FIG. 1 is not intended to be limiting. An individual sensor 142 may be located at or near (a body part of) subject 106, embedded and/or integrated in respiratory medicament delivery device 11, and/or at other locations. Resulting output signals or conveyed information from one or more sensors 142 may be transmitted to processor 110, user interface 120, electronic storage 130, and/or other components of system 10. Transmission may be wired and/or wireless.

The one or more sensors 142 may be configured to generate output signals in an ongoing manner, *e.g.* before, during, and/or after delivery of medicament. This may include generating signals intermittently, periodically (*e.g.* at a sampling rate), continuously, continually, at varying intervals, and/or in other ways that are ongoing. The sampling rate may be about 10⁻⁹ second, about 10⁻⁸ second, about 10⁻⁷ second, 10⁻⁶ second, 10⁻⁵ second, 10⁻⁴ second, 0.001 second, 0.01 second, 0.1 second, 1 second, about 10 seconds, about 1 minute, and/or other sampling rates. It is noted that multiple individual sensors 142 may operate using different sampling rates, as appropriate for the particular output signals and/or (frequencies related to particular) parameters and/or characteristics derived therefrom. For example, in some embodiments, the generated output signals may be considered as a vector of output signals, such that a vector includes multiple samples of information conveyed related to one or more parameters and/or characteristics. A particular parameter or characteristic determined in an ongoing manner from a vector of output signals may be considered as a vector of that particular parameter or characteristic.

In some implementations, sensor 142 may be a MEMS microphone configured and/or arranged to measure ultrasonic energy transferred from any flat and/or curved surface within respiratory medicament delivery device 11 and/or any such exterior surface of respiratory medicament delivery device 11.

In some implementations, sensor 142 may be configured to generate output signals conveying measurements related to gas parameters of respiratory airflow, parameters related to airway mechanics, parameters related to respiratory timing, and/or other parameters. Gas parameters may include flow, (airway) pressure, humidity, velocity, acceleration, and/or other gas parameters. Output signals may convey measurements related to respiratory parameters. Sensor 142 may be in fluid communication with conduit 180 and/or mouthpiece 184. Sensor 142 may generate output signals related to physiological parameters pertaining to subject 106. Parameters may be associated with the state and/or condition of an airway of subject 106, the breathing of subject 106, the gas breathed by subject 106, the composition of the gas breathed by subject 106, the delivery of the gas to the airway of subject 106, and/or a respiratory effort by the subject.

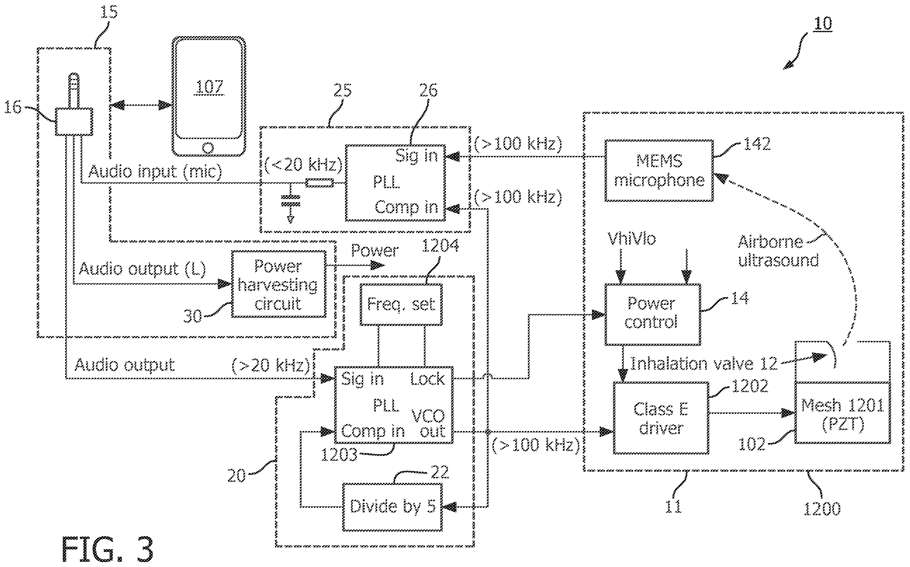

By way of illustration, FIG. 3 schematically illustrates a system 10 that includes respiratory medicament delivery device 11 (as depicted including a mesh nebulizer 1200 and an inhalation valve 12), (MEMS) microphone 142, a power subsystem 15 (as depicted including a power harvesting circuit 30 and a connector 16), a sound input conversion subsystem 20, a sound output conversion subsystem 25, and/or other components. Components of system 10 may be included, omitted, and/or replaced by other components independently of each other. In some implementations, system 10 may include fewer components and/or different components than depicted in FIG. 3. By way of non-limiting example, in some implementations, system 10 may not include a sound output conversion subsystem, and/or not include a power harvesting circuit.

Referring to FIG. 3, mesh nebulizer 1200 may include one or more of a vibrating element 102 (as depicted including a mesh 1201), a class E driver 1202, power control 14, and/or other components.

Power subsystem 15 may include one or more of power harvesting circuit 30, connector 16, and/or other components. In some implementations, power subsystem 15 may include one or more cable to implement connections described in this disclosure and/or illustrated in the figures. Connector 16 may include one or more of a cable, a headphone jack (including but not limited to a 3.5 mm jack), an earphone jack, a microphone connector, a telephone connector, a TS connector, a TRS connector, a TRRS connector, an audio jack, a docking connector (including but not limited to an iPhone™ docking connector), a docking station (including but not limited to an iPhone™ docking station), a universal serial bus (USB) connector, an XLR connector, and/or another type of connector. Power subsystem 15 may be configured to receive an input signal and provide power based on and/or from the received input signal. In some implementations, the received input signal may include one or more audio signals. In some implementations, the input signal may be received from a computing device, including but not limited to computing device 107. In some implementations, connector 16 may be used simultaneously for input and output. In some implementations, system 10 may include a cable to connect power subsystem 15 and computing device 107.

In some implementations, power harvesting circuit 30 may be configured to harvest power from one or more signals of power subsystem 15. For example, power harvesting circuit may be configured to provide power from one of the two audio signals provided through connector 16 (as depicted by label "audio output (L)"). In some implementations, power harvesting circuit 30 may provide power to a nebulizer and/or droplet generator that is based on Fourier-horn technology. In some implementations, power subsystem 15 may be configured to provide power through a USB power port (as depicted by label "USB power out").

Sound input conversion subsystem 20 may include one or more of a PLL 1203, a frequency set 1204, a divider circuit 22, and/or other components. Sound input conversion subsystem may be configured to receive an input signal (by way of non-limiting example, an audio input signal). Sound input conversion subsystem 20 may be configured to convert an input signal (*e.g.* an input signal received from and/or through power subsystem 15) to an ultrasonic output signal. PLL 1203 may include inputs "signal in" (or sig in) and "comparator in" (or comp in) and outputs "VCO-out" and "lock," all of which may be standard for PLLs. Output "VCO-out" may loop back to input "comparator in" through divider circuit 22 (as depicted by way of non-limiting example, a divide-by-five circuit). PLL 1203 may be configured to provide a driving frequency for mesh 1201 and/or a vibrating element of respiratory medicament delivery device 11 that is five times higher (and thus ultrasonic) than the input frequency (through a suitable driver such as Class E driver 1202). The factor for divider circuit 22 is not limited to 5; this is merely exemplary to illustrate the conversion of an audible (or barely audible) input signal of about 20 kHz to an ultrasonic signal of about 100 kHz, used to drive mesh 1201.

In some implementations, PLL 1203 may be configured to adjust the driving frequency based on a phase difference between the ultrasonic energy measured through microphone 142 and the signal/frequency used to drive mesh 1201 (*e.g.* from output VCO-out). Note that microphone 142 may need to be positioned such that contact with aerosol is avoided or minimized, *e.g.* by placing microphone 142 at a suitable harmonic distance (*i.e.* one or more cycles) from mesh 1201. Note that the signal from output VCO-out may be a square wave, whereas the signal from mesh 1201 may be a sinusoid, though their frequencies are necessarily the same.

If and/or when the operating frequency of mesh 1201 changes away from resonance, the energy emitted by mesh 1201 will decrease in amplitude (due to the impedance curve of the element used to drive mesh 1201), effectively increasing the phase difference. In response, PLL 1203 may adjust its output frequency to counteract this condition. In some implementations, mesh nebulizer 1200 may include frequency set 1204 configured to manually and/or programmably control PLL 1203.

PLL 1203 may be configured, once it is locked, to adjust operating conditions such that the phase difference is minimized, and the energy amplitude (at least locally) maximized. The features described in this disclosure may be used to detect conditions including sputter, end of treatment, and/or other conditions.

Referring to FIG. 3, in some implementations, system 10 includes an inhalation valve 12 (*e.g.* an inhalation flap valve). Inhalation valve 12 may be configured to move responsive to a flow of air and/or gas. Inhalation valve 12 may be included in a flow path of respiratory medicament delivery device 11. For example, inhalation valve 12 may be configured to move responsive to respiration by subject 106. For example, inhalation valve 12 may open responsive to inhalation by subject 106 and/or close responsive to exhalation by subject 106, thus forming a basis for breath-actuation. Inhalation valve 12 may be configured and/or arranged to reduce the ultrasonic energy received by microphone 142.

By virtue of inhalation valve 12, the magnitude of the measured ultrasonic energy of respiratory medicament delivery device 11 may vary with a position of inhalation valve 12 (*e.g.* being open or closed). The measured magnitude may be used to control operation of respiratory medicament delivery device 11 and/or monitor respiratory parameters (*e.g.* as indicative of patient adherence). For example, such information may be used to control delivery of medicament to subject 106. This may, *e.g.,* prevent wasting medicament during exhalation. Referring to FIG. 3, in some implementations, system 10 may be configured to adjust the operating frequency (*e.g.* off-resonance) and/or reduce (drive) power responsive to the inhalation flap valve being closed. As a result, aerosol production may be reduced and/or halted; at least until the inhalation flap valve is opened upon the next inhalation by subject 106. Such a mode of operation may be referred to as breath-actuated. Variations using an exhalation (flap) valve are considered within the scope of this disclosure.

Sound output conversion subsystem 25 may include one or more of a PLL 26, and/or other components (*e.g.* as depicted in FIG. 3). PLL 26 may be structurally similar to other PLLs depicted in FIG. 3. Sound output conversion subsystem 25 may be configured to receive an ultrasonic signal from mesh nebulizer 1200 and/or MEMS microphone 142. By virtue of input "comparator in" (or comp in) being provided by the same divider circuit 22 as described elsewhere in relation to sound input conversion subsystem 20, the output signal of sound output conversion subsystem 25 may be at about 20 kHz (assuming a 100 kHz operation frequency for mesh 1202 for illustrative purposes), and may be suitable for a microphone input of a computing device 107 (for example through connector 16). In some implementations, for example in cases where respiratory medicament delivery device 11 is a type of respiratory medicament delivery device that emits energy at audible or sub-20 kHz frequencies, system 10 may not include a sound output conversion subsystem 25. For example, such systems may include an audio amplifier (not depicted) to connect the output generated by microphone 142 to computing device 107.

Referring to FIG. 3, in some implementations, system 10 may include power control 14. Power control 14 may be controlled based on, at least in part, an output from PLL 1203, such as, *e.g.,* the lock output. When PLL 1203 is locked, *e.g.* when inhalation valve 12 is open, power control 14 may be configured to control Class E driver 1202 to use a high power setting that is sufficient for system 10 to produce aerosol. When PLL 1203 is not locked, a low power setting may be used. Alternatively, and/or simultaneously, alternative implementations to control nebulization are contemplated within the scope of this disclosure. Note that the low power setting may need to be sufficiently powerful such that, once inhalation valve 12 is opened again, PLL 1203 can once again lock. Power control 14 may be configured to provide gain control for Class E Driver 1202, and thus for mesh 1201. Note that a breath-actuated mode of operation as described herein may be used for different types of respiratory medicament delivery devices.

Information derived from output signals generated by MEMS microphone 142 and/or other (energy) measurements may be used to determine device actuation, respiratory rate, inhalation period, exhalation period, flow rate, strength of inhalation by a patient, amount of drug delivered, number of drug delivery sessions in a day or week, *etc.* Based on a comparison of such measured and/or determined information and the recommended treatment for a subject, a level of patient adherence may be determined. Combinations of different types of measured and/or determined information are contemplated within the scope of this disclosure. For example, device actuation information may be combined with patient-specific respiratory information to determine a metric for patient adherence. Device actuation and/or detection of device actuation may characterize operating of respiratory medicament delivery device 11 and/or any component thereof.

Returning to FIG. 1, electronic storage 130 of system 10 comprises electronic storage media that electronically stores information. The electronic storage media of electronic storage 130 may include one or both of system storage that is provided integrally (*i.e.,* substantially non-removable) with system 10 and/or removable storage that is removably connectable to system 10 via, for example, a port (*e.g.,* a USB port, a FireWire port, *etc*.) or a drive (*e.g.,* a disk drive, *etc*.). Electronic storage 130 may include one or more of optically readable storage media (*e.g.,* optical disks, *etc*.), magnetically readable storage media (*e.g.,* magnetic tape, magnetic hard drive, floppy drive, *etc*.), electrical charge-based storage media (*e.g.,* EPROM, EEPROM, RAM, *etc*.), solid-state storage media (*e.g.,* flash drive, *etc*.), and/or other electronically readable storage media. Electronic storage 130 may store software algorithms, information determined by processor 110, information received via user interface 120, and/or other information that enables system 10 to function properly. For example, electronic storage 130 may record or store vectors of parameters based on the generated output signals, and/or other parameters (as discussed elsewhere herein), and/or other information. Electronic storage 130 may be a separate component within system 10, or electronic storage 130 may be provided integrally with one or more other components of system 10 (*e.g.,* processor 110).

User interface 120 of system 10 in FIG. 1 is configured to provide an interface between system 10 and a user (*e.g.,* a user 108, subject 106, a caregiver, a therapy decision-maker, *etc*.) through which the user can provide information to and receive information from system 10. This enables data, results, and/or instructions and any other communicable items, collectively referred to as "information," to be communicated between the user and system 10. An example of information that may be conveyed by user 108 to system 10 is patient-specific adherence information. An example of information that may be conveyed to user 108 is a report detailing adherence information for subject 106. Examples of interface devices suitable for inclusion in user interface 120 include a keypad, buttons, switches, a keyboard, knobs, levers, a display screen, a touch screen, speakers, a microphone, an indicator light, an audible alarm, and a printer. Information may be provided to user 108 or subject 106 by user interface 120 in the form of auditory signals, visual signals, tactile signals, and/or other sensory signals.

It is to be understood that other communication techniques, either hardwired or wireless, are also contemplated herein as user interface 120. For example, in one embodiment, user interface 120 may be integrated with a removable storage interface provided by electronic storage 130. In this example, information is loaded into system 10 from removable storage (*e.g.,* a smart card, a flash drive, a removable disk, *etc*.) that enables the user(s) to customize system 10. Other exemplary input devices and techniques adapted for use with system 10 as user interface 120 include, but are not limited to, an RS-232 port, RF link, an IR link, modem (telephone, cable, Ethernet, internet or other). In short, any technique for communicating information with system 10 is contemplated as user interface 120.

Processor 110 of system 10 in FIG. 1 is configured to provide information processing capabilities in system 10. As such, processor 110 includes one or more of a digital processor, an analog processor, a digital circuit designed to process information, an analog circuit designed to process information, and/or other mechanisms for electronically processing information. Although processor 110 is shown in FIG. 1 as a single entity, this is for illustrative purposes only. In some embodiments, processor 110 includes a plurality of processing units.

As is shown in FIG. 1, processor 110 is configured to execute one or more computer program modules. The one or more computer program modules include one or more of parameter determination module 111, control module 112, and/or other modules. Processor 110 may be configured to execute modules 111-113 by software; hardware; firmware; some combination of software, hardware, and/or firmware; and/or other mechanisms for configuring processing capabilities on processor 110. In some embodiments, processor 110 and/or one or more computer program modules may be implemented on computing device 107. For example, one or more computer program modules may be part of a software application (or "app") being executed on computing device 107.

It should be appreciated that although modules 111-113 are illustrated in FIG. 1 as being co-located within a single processing unit, in embodiments in which processor 110 includes multiple processing units, one or more of modules 111-113 may be located remotely from the other modules. The description of the functionality provided by the different modules 111-113 described herein is for illustrative purposes, and is not intended to be limiting, as any of modules 111-113 may provide more or less functionality than is described. For example, one or more of modules 111-113 may be eliminated, and some or all of its functionality may be incorporated, shared, integrated into, and/or otherwise provided by other ones of modules 111-113. Note that processor 110 may be configured to execute one or more additional modules that may perform some or all of the functionality attributed below to one of modules 111-113.

Parameter determination module 111 of system 10 in FIG. 1 is configured to determine one or more parameters from output signals generated by sensor(s) 142. The one or more parameters may include a first spectral parameter, one or more operational parameters, respiratory timing parameters, and/or other parameters. The first spectral parameter may indicate (magnitude of) energy amplitude in a first frequency band. For example, the first spectral parameter may indicate the amplitude of the ultrasonic energy received by microphone 142 as described elsewhere herein. The first spectral parameter may characterize operation of respiratory medicament delivery device 11. In some embodiments, parameter determination module 111 is configured to determine additional spectral parameters in a manner similar to the first spectral parameter, though, *e.g.,* corresponding to other frequency bands. As used herein, the term "magnitude" may be used to refer to the energy amplitude at a particular frequency and/or within a particular range of frequencies.

Operation of parameter determination module 111 may be performed in an ongoing manner, for example at a particular sampling rate. The one or more parameters may be determined at different locations and/or positions within system 10 or near subject 106. In some embodiments, parameter determination module 111 may derive vectors of parameters in an ongoing manner during a period of monitoring subject 106. The vectors of the parameters may be based on vectors of generated output signals and/or other (vectors of) determined parameters. Parameter determination module 111 may be configured to determine an operational parameter that indicates operation of respiratory medicament delivery device 11, including but not limited to duration of treatment, frequency of treatment (*e.g.* per week), and/or other parameters.

Control module 112 is configured to control respiratory medicament delivery device 11 during operation. Operation of control module 112 may be based on one or more parameters determined by parameter determination module 111. Control by control module 112 may include adjustments, *e.g.* of the operating frequency, drive power, and/or any other adjustable operating conditions as described herein. Adjustments may be based on determined (spectral) parameters and/or generated output signals. Adjustments may be made such that a particular determined parameter, *e.g.* the first spectral parameter, is maintained at or above at or above a predetermined threshold level. In some implementations, such a threshold is predetermined at a percentage of the known maximum for the particular determined parameter. The predetermined percentage may be about 80%, about 90%, about 95%, about 97%, about 98%, about 99%, and/or another percentage. Adjustments may be made in an ongoing manner, for example at a particular sampling rate. Adjustments may be made in real-time or near-real-time. The rate of adjustment may be milliseconds, 0.5 second, 1 second, 2 seconds, 5 seconds, 10 seconds, 20 seconds, and/or another appropriate rate.

Adherence module 113 is configured to determine an adherence metric and/or an adherence parameter for subject 106. In some implementations, adherence module 113 may be executed on computing device 107. The adherence metric and/or adherence parameter may be based on one or more parameters determined by parameter determination module 111. For example, a particular adherence metric may be based on a combination of device actuation information and respiratory information/timing. An adherence metric and/or adherence parameter may for example be expressed as a percentage of perfect compliance with the recommended treatment. For example, if a particular patient scored a 90% adherence, such a score that may be considered by a care giver in determining a course of action. Alternatively, if a particular patient scored a low percentage of adherence, such a score may be considered relevant before the particular drug is deemed ineffective for that particular patient. Low scores may prompt a change in the chosen type of drug delivery device.

In some implementations, determinations by adherence module 113 may be analyzed, processed, gathered, aggregated, and/or transmitted by adherence module 113 and/or computing device 107. In some implementations, determinations by adherence module 113 and/or information based thereon may be transmitted by computing device 107 to an external server that may be associated with a caregiver, a doctor, a hospital, and/or other pertinent parties that are authorized to receive such information.

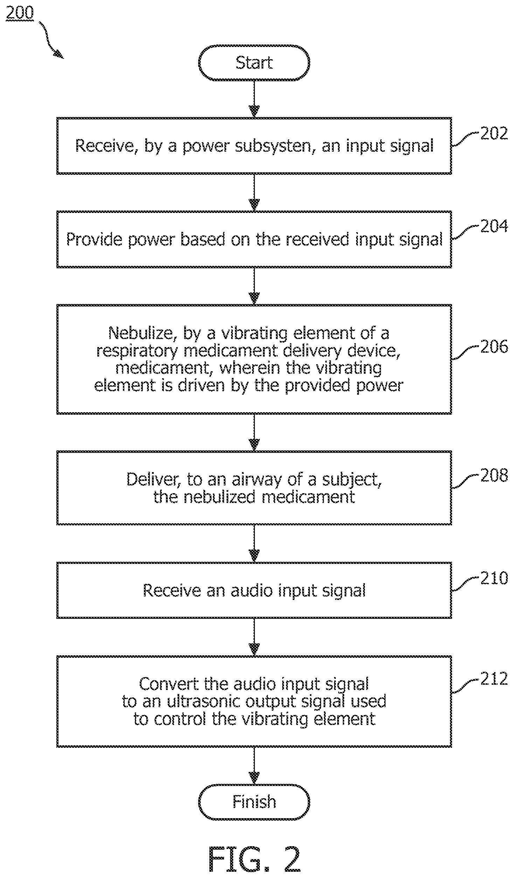

FIG. 2 illustrates a method 200 to deliver medicament to a subject, not part of the invention. The operations of method 200 presented below are intended to be illustrative. In certain examples, method 200 may be accomplished with one or more additional operations not described, and/or without one or more of the operations discussed. Additionally, the order in which the operations of method 200 are illustrated in FIG. 2 and described below is not intended to be limiting.

In certain examples, method 200 may be implemented in one or more processing devices (*e.g.,* a digital processor, an analog processor, a digital circuit designed to process information, an analog circuit designed to process information, and/or other mechanisms for electronically processing information). The one or more processing devices may include one or more devices executing some or all of the operations of method 200 in response to instructions stored electronically on an electronic storage medium. The one or more processing devices may include one or more devices configured through hardware, firmware, and/or software to be specifically designed for execution of one or more of the operations of method 200.

At an operation 202, an input signal is received by a power subsystem. In some embodiments, operation 202 is performed by a power subsystem the same as or similar to power subsystem 15 (shown in FIG. 3 and described herein).

At an operation 204, power is provided based on the received input signal. In some embodiments, operation 204 is performed by a power subsystem the same as or similar to power subsystem 15 (shown in FIG. 3 and described herein).

At an operation 206, medicament is nebulized by a vibrating element of a respiratory medicament delivery device. The vibrating element is driven by the provided power. In some examples, operation 206 is performed by a piezoelectric element the same as or similar to piezoelectric element 102 (shown in FIG. 3 and described herein).

At an operation 208, the nebulized medicament is delivered to an airway of a subject. In some examples, operation 208 is performed by a respiratory medicament delivery device the same as or similar to respiratory medicament delivery device 11 (shown in FIG. 1 and described herein).

At an operation 210, an audio input signal is received. In some examples, operation 210 is performed by a sound input conversion subsystem the same as or similar to sound input conversion subsystem 20 (shown in FIG. 1 and described herein).

At an operation 212, the audio input signal is converted to an ultrasonic output signal. The step of nebulizing medicament is performed by controlling the vibrating element based on the ultrasonic output signal. In some examples, operation 212 is performed by a sound input conversion subsystem the same as or similar to sound input conversion subsystem 20 (shown in FIG. 1 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although this description includes details for the purpose of illustration based on what is currently considered to be the most practical and preferred embodiments, it is to be understood that such detail is solely for that purpose and that the disclosure is not limited to the disclosed embodiments, but, on the contrary, is intended to cover modifications and equivalent arrangements that are within the scope of the appended claims. For example, it is to be understood that, to the extent possible, one or more features of any embodiment are contemplated to be combined with one or more features of any other embodiment.

## Claims

1. A system (10) configured to deliver medicament to a subject (106), the system comprising:
a power subsystem (15) configured to receive an input signal and provide power based on the received input signal;
a respiratory medicament delivery device (11) that includes a vibrating element (102) that is driven by power provided by the power subsystem, wherein the respiratory medicament delivery device is configured to deliver medicament, responsive to nebulization of the medicament by the vibrating element, to an airway of a subject (106); **characterized by**;
a sound input conversion subsystem (20) configured to receive an audio input signal having a first frequency, wherein the sound input conversion subsystem is configured to convert the audio input signal to an ultrasonic output signal having a second frequency in dependence of the first frequency,
wherein the vibrating element is controlled based on the ultrasonic output signal,
wherein mechanical movement of the vibrating element causes emission of ultrasonic energy during operation of the respiratory medicament delivery device, the system further comprising:
a sensor (142) configured to generate output signals representing one or more characteristics of the ultrasonic energy emitted during operation of the respiratory medicament delivery device; and
a sound output conversion subsystem (25) configured to receive the generated output signals, wherein the sound output conversion subsystem is configured to convert the generated output signals into an audio output signal, and wherein the sound output conversion subsystem is further configured to provide the audio output signal to a computing device (107).

2. The system of claim 1, wherein the input signal includes the audio input signal.

3. The system of claim 1, wherein the audio input signal is received from a connector(16) of a computing device (107), wherein the power subsystem is configured to receive the input signal from the connector of the computing device, wherein the power subsystem includes a power harvesting circuit (30) configured to harvest power from the input signal, the system further comprising:
one or more processors (110) configured to execute computer program modules, the computer program modules comprising:
a parameter determination module (111) configured to determine, based on the audio output signal, a spectral parameter that indicates energy amplitude of ultrasonic energy emitted by the respiratory medicament delivery device, such that the first spectral parameter characterizes operation of the respiratory medicament delivery device;
a control module (112) configured to control the audio input signal based on the spectral parameter; and
an adherence module (113) configured to determine an adherence metric based on the spectral parameter.

4. The system of claim 3, wherein one or more of the parameter determination module, the control module, and/or the adherence module is included in the computing device.

5. The system of claim 1, wherein the computing device (107) comprises a desktop computer, a laptop computer, a handheld computer, a NetBook, a tablet, a smartphone, a wireless telephone, a portable electronic device, a handheld communication device, a digital camera, and a gaming console,

6. The system of claim 1, wherein the computing device (107) is configured to;
provide power to respiratory medicament delivery device (11), or;
provide one or more control functions for the operation of respiratory medicament delivery device (11), or;
provide adherence monitoring for respiratory medicament delivery device (11).

## Patentansprüche

1. System (10), das zur Abgabe eines Medikaments an ein Objekt (106) konfiguriert ist, wobei das System umfasst:
ein Leistungssubsystem (15), welches so konfiguriert ist, dass es ein Eingangssignal empfängt und aufgrund des empfangenen Eingangssignals Leistung bereitstellt;
eine Atemwegsmedikament-Abgabevorrichtung (11), die ein Vibrationselement (102) enthält, das durch von dem Leistungssubsystem bereitgestellte Leistung angetrieben wird, wobei die Atemwegsmedikament-Abgabevorrichtung so konfiguriert ist, dass sie in Reaktion auf eine Vernebelung des Medikaments durch das Vibrationselement das Medikament an einen Atemweg eines Objekts (106) abgibt; **gekennzeichnet durch**:
ein Sound-Input-Umwandlungssubsystem (20), das so konfiguriert ist, dass es ein Audioeingangssignal mit einer ersten Frequenz empfängt, wobei das Sound-Input-Umwandlungssubsystem so konfiguriert ist, dass es das Audioeingangssignal in ein Ultraschallausgangssignal mit einer zweiten Frequenz in Abhängigkeit der ersten Frequenz umwandelt,
wobei das Vibrationselement aufgrund des Ultraschallausgangssignals gesteuert wird,
wobei eine mechanische Bewegung des Vibrationselements bei Betrieb der Atemwegsmedikament-Abgabevorrichtung eine Ultraschallenergieemission bewirkt, wobei das System weiterhin umfasst:
einen Sensor (142), der so konfiguriert ist, dass er Ausgangssignale erzeugt, die eine oder mehrere Charakteristiken der während des Betriebs der Atemwegsmedikament-Abgabevorrichtung emittierten Ultraschallenergie darstellen; sowie
ein Sound-Output-Umwandlungssubsystem (25), das so konfiguriert ist, dass es die erzeugten Ausgangssignale empfängt, wobei das Sound-Output-Umwandlungssubsystem so konfiguriert ist, dass es die erzeugten Ausgangssignale in ein Audioausgangssignal umwandelt, und wobei das Sound-Output-Umwandlungssystem weiterhin so konfiguriert ist, dass es das Audioausgangssignal einer Rechenvorrichtung (107) zuführt.

2. System nach Anspruch 1, wobei das Eingangssignal das Audioeingangssignal enthält.

3. System nach Anspruch 1, wobei das Audioeingangssignal von einem Anschluss (16) einer Rechenvorrichtung (107) empfangen wird, wobei das Leistungssubsystem so konfiguriert ist, dass es das Eingangssignal von dem Anschluss der Rechenvorrichtung empfängt, wobei das Leistungssubsystem eine Leistungsgewinnungsschaltung (30) enthält, die so konfiguriert ist, dass sie Leistung aus dem Eingangssignal gewinnt, wobei das System weiterhin umfasst:
einen oder mehrere Prozessoren (110), die so konfiguriert sind, dass sie Computerprogrammodule ausführen, wobei die Computerprogrammodule umfassen:
ein Parameterermittlungsmodul (111), das so konfiguriert ist, dass es aufgrund des Audioausgangssignals einen Spektralparameter ermittelt, der die Energieamplitude der von der Atemwegsmedikament-Abgabevorrichtung emittierten Ultraschallenergie anzeigt, so dass der erste Spektralparameter den Betrieb der Atemwegsmedikament-Abgabevorrichtung charakterisiert;
ein Steuermodul (112), das so konfiguriert ist, dass es das Audioeingangssignal aufgrund des Spektralparameters steuert; sowie
ein Adhärenz-Modul (113), das so konfiguriert ist, dass es eine Adhärenz-Metrik aufgrund des Spektralparameters ermittelt.

4. System nach Anspruch 3, wobei ein oder mehrere des Parameterermittlungsmoduls, des Steuermoduls und/oder des Adhärenzmoduls in der Rechenvorrichtung enthalten sind/ist.

5. System nach Anspruch 1, wobei die Rechenvorrichtung (107) einen Desktop-Computer, einen Laptop-Computer, einen Handheld-Computer, ein NetBook, ein Tablet, ein Smartphone, ein Drahtlostelefon, ein portables Elektronikgerät, ein Handheld-Kommunikationsgerät, eine Digitalcamera und eine Spielconsole umfasst.

6. System nach Anspruch 1, wobei die Rechenvorrichtung (107) so konfiguriert ist, dass sie:
der Atemwegsmedikament-Abgabevorrichtung (11) Leistung zuführt, oder;
eine oder mehrere Steuerfunktionen für den Betrieb der Atemwegsmedikament-Abgabevorrichtung (11) bereitstellt, oder:
eine Adhärenz-Überwachung für die Atemwegsmedikament-Abgabevorrichtung (11) bereitstellt.

## Revendications

1. Système (10) configuré pour administrer un médicament à un sujet (106), le système comprenant :
un sous-système d'alimentation (15) configuré pour recevoir un signal d'entrée et fournir de l'énergie sur la base du signal d'entrée reçu ;
un dispositif d'administration de médicament respiratoire (11) qui comprend un élément vibrant (102) qui est entraîné par l'énergie fournie par le sous-système d'alimentation, dans lequel le dispositif d'administration de médicament respiratoire est configuré pour administrer le médicament, à la suite de la nébulisation du médicament par l'élément vibrant, à une voie aérienne d'un sujet (106), **caractérisé par** :
un sous-système de conversion d'entrée sonore (20) configuré pour recevoir un signal d'entrée audio ayant une première fréquence, dans lequel le sous-système de conversion d'entrée sonore est configuré pour convertir le signal d'entrée audio en un signal de sortie ultrasonique ayant une seconde fréquence en fonction de la première fréquence,
dans lequel l'élément vibrant est commandé sur la base du signal de sortie ultrasonique,
dans lequel le mouvement mécanique de l'élément vibrant provoque l'émission d'énergie ultrasonique au cours du fonctionnement du dispositif d'administration de médicament respiratoire, le système comprenant en outre :
un capteur (142) configuré pour générer des signaux de sortie représentant une ou plusieurs caractéristiques de l'énergie ultrasonique émise au cours du fonctionnement du dispositif d'administration de médicament respiratoire ; et
un sous-système de conversion de sortie sonore (25) configuré pour recevoir les signaux de sortie générés, dans lequel le sous-système de conversion de sortie sonore est configuré pour convertir les signaux de sortie générés en un signal de sortie audio et dans lequel le sous-système de conversion de sortie sonore est en outre configuré pour fournir le signal de sortie audio à un ordinateur (107).

2. Système selon la revendication 1, dans lequel le signal d'entrée comprend le signal d'entrée audio.

3. Système selon la revendication 1, dans lequel le signal d'entrée audio est reçu d'un connecteur (16) d'un ordinateur (107), dans lequel le sous-système d'alimentation est configuré pour recevoir le signal d'entrée du connecteur de l'ordinateur, dans lequel le sous-système d'alimentation comprend un circuit collecteur d'énergie (30) configuré pour recueillir de l'énergie du signal d'entrée, le système comprenant en outre :
un ou plusieurs processeurs (110) configurés pour exécuter des modules de programme informatique, les modules de programme informatique comprenant :
un module de détermination de paramètres (111) configuré pour déterminer, sur la base du signal de sortie audio, un paramètre spectral qui indique l'amplitude d'énergie de l'énergie ultrasonique émise par le dispositif d'administration de médicament respiratoire de sorte que le premier paramètre spectral caractérise le fonctionnement du dispositif d'administration de médicament respiratoire ;
un module de commande (112) configuré pour commander le signal d'entrée audio sur la base du paramètre spectral ; et
un module d'adhérence (113) configuré pour déterminer une métrique d'adhérence basée sur le paramètre spectral.

4. Système selon la revendication 3, dans lequel un ou plusieurs du module de détermination de paramètres, du module de commande et/ou du module d'adhérence est ou sont inclus dans l'ordinateur.

5. Système selon la revendication 1, dans lequel l'ordinateur (107) comprend un ordinateur de bureau, un ordinateur portable, un ordinateur manuel, un NetBook, une tablette, un smartphone, un téléphone sans fil, un dispositif électronique portable, un dispositif de communication portatif, une caméra numérique et une console de jeux.

6. Système selon la revendication 1, dans lequel l'ordinateur (107) est configuré pour :
fournir de l'énergie à un dispositif d'administration de médicament respiratoire (11), ou ;
fournir une ou plusieurs fonctions de commande pour le fonctionnement du dispositif d'administration de médicament respiratoire (11), ou ;
fournir une surveillance d'adhérence pour le dispositif d'administration de médicament respiratoire (11).
